# EUROPEAN PATENT SPECIFICATION

(11) **EP 2 533 773 B1**
(45) Date of publication and mention of the grant of the patent: **19.08.2015**
(21) Application number: 11773727.0
(22) Date of filing: 21.10.2011
(51) Int. Cl.: A61K 9/70, A61L 15/44

(54) **TOPICAL DERMAL DELIVERY DEVICE FOR NITRIC OXIDE DELIVERY**
VORRICHTUNG ZUR TOPISCHEN DERMALEN ABGABE VON STICKOXID
DISPOSITIF D'ADMINISTRATION PAR VOIE CUTANÉE TOPIQUE D'OXYDE NITRIQUE

(30) Priority: 21.10.2010 US 405248 P
(43) Date of publication of application: 19.12.2012
(73) Proprietor: KIPAX AB, 251 10 Helsingborg (SE)
(72) Inventor: STHENGEL, Elisabeth, S-25438 Helsingborg (SE); NGO, See Man, S-28133 Hässleholm (SE); LINDGREN, Lars, S-25440 Helsingborg (SE)
(74) Representative: KIPA AB
(86) International application number: PCT/EP2011/068489
(87) International publication number: WO 2012/052561

(56) References cited:
- WO-A1-2008/116497
- US-A- 5 648 101

## Description

### Field of the Invention

The invention relates to devices, methods, and systems for generating nitric oxide gas and to the use of non-implantable, delivery devices for the administration of essentially pure nitric oxide gas (NO) to prevent, treat or augment preventions or treatments for conditions including pain, wounds, infection, circulatory impairments, and cardiovascular disease. The nitric oxide delivery devices may also be used for cosmetic purposes. The therapeutic or cosmetic effects are accomplished by activating and applying a device, such as a patch or balloon catheter, containing a preparation that is stable during storage and that is easily activated to release an amount of essentially pure nitric oxide gas over an extend period of time to a human and/or animal subject.

### Background of the Invention

Nitric oxide is a small, uncharged molecule that passes freely across cell membranes and acts as a messenger within and among cells. It is produced endogenously by nitric oxide producing enzymes (Nitric oxide-synthases, NOS) located in endothelial cells, neuronal cells, or macrophages. Nitric oxide is a highly reactive molecule with a half-life ranging from milliseconds to seconds and scavenging reactions further limit the distance over which it can diffuse. As a result, nitric oxide acts near its source of production and its direct effects are transient and local.

Various compounds and devices for topical treatment of numerous conditions are known. WO 2008/116497 and 2008/116925 relate to topical nitric oxide delivery systems and EP 1871433 disclosed a device for cosmetic treatment with nitric oxide, all of which are by the applicant of the present application and are incorporated herein by reference in their entirety and for all purposes.

US 5,648,101, incorporated herein by reference in its entirety, discloses a method for delivering NO gas to a desired location on or in the body of a subject by way any of a number of forms including tablets, creams, lotions, sprays and patches. The chemical reaction used to generate NO gas involves equimolar amounts of a soluble reducing salt such as ferrous sulfate and an equimolar amount nitrite salt in the presence of an aqueous organic acid. The patch delivery system comprises a multi-layer macroporous or foraminous gauze or cellulosic matrix impregnated with ferrous sulfate solution and covered with an outer water resistant foil web. Before application to the skin, sodium nitrite in water is added to the active area of the patch and after activation the patch delivers NO for no more than 1 hour. The active area of the patch is not covered and NO is released from the gauze or matrix and diffuses directly to the skin. Consequently, only the total amount of NO released from the patch can be controlled. Neither the rate nor the duration of NO release is controlled.

Unlike the patch disclosed in US 5,648,101, a nitric oxide delivery device according to the present invention is able to provide a controlled release of NO over a long duration. This is accomplished by providing a NO permeable membrane having a controlled NO permeability and also by providing reducing, nitrosyl complex forming agent in an excess over NO produced so that a reservoir of complexed NO is formed that releases NO from the device with predictable kinetics.

WO 02/17881 A2, incorporated by reference in its entirety, discloses a delivery system comprising a pharmaceutically active agent and an acidified nitrite to produce nitric oxide at the skin surface. US/2005/0142218A1, incorporated by reference in its entirety, discloses a composition containing nitrite sources and an organic acid acidifying agent to treat skin ischemia. The production of NO for both of the above occurs via reaction between nitrite ion and an aqueous acid to produce NO and NO₂, the latter of which is a toxic irritant. Neither the rate nor the duration of NO release is controlled.

Unlike the devices and methods disclosed in US 5,648,101, WO 02/17881 A2, and US/2005/0142218 A1, the present invention provides for controlling not only the total amount of NO released, but also the rates at which NO is produced within the devices and rates and durations of NO release from the devices. Prior art devices do not enable the design and production of NO delivery devices having a predetermined profile of NO release selected from a range of NO release, or delivery, profiles. Additionally, devices and methods according to the present invention prevent toxic or irritating chemicals such as ascorbyl radical and NO₂ from reaching the treatment site and may be configured to use the formation of a colored transient NO complex as an indicator to monitor NO production and depletion. The present invention provides for oxygen that inactivate reducing agents during storage and cause the formation of NO₂ after device activation are avoided and embodiments in which a semipermeable membrane allows release of NO but not irritating byproducts.

### Summary of the Invention

The present invention provides for nitric oxide (NO) delivery devices, methods, systems, and methods for their production. The devices, methods, and systems comprise a NO precursor and a reducing agent capable of forming a transient complex with NO contained within an enclosure. The NO precursor is separated from the reducing and complexing agent by a barrier. Nitric oxide production and release is activated by breaking or removing the barrier to allow NO precursor to mix with the reducing/complexing agent. One side of the enclosure is configured to contact treatment site of a human or animal subject and comprises a membrane having a controlled NO permeability.

The reducing/complexing agent is preferably present in an amount that is a molar excess over the NO produced from the NO precursor and may additionally serve as an indicator by changing color within the device when complexed with NO. The NO permeable membrane is preferably covered by an oxygen impermeable removable release liner to be removed immediately prior to application of the device to a treatment site. The device may be manufactured in an oxygen limited environment and comprise materials that prevent the penetration of atmospheric oxygen into the device. The amount of NO produced and released, the duration of NO production and release, and the kinetic profile of NO delivery from the device to the subject may be controlled by the selection of one or more of: the relative and absolute amounts of nitrite or other NO precursor and reducing/complexing agent, the NO permeability of the membrane facing a treatment site, the volume of the device, and the surface area of the NO permeable membrane.

Devices according to the invention may have a variety of forms including cutaneous patches, socks, gloves, condoms, caps and balloon catheters (e.g. Foley catheters) and may be used in methods of preventing or treating pain from any cause, neuropathies, healing of wounds, scar formation, infections of all kinds, circulatory impairments including erectile dysfunction, warts, and other hyperproliferations of cells. The devices are cosmetically useful for treating, for example, hair loss, scaring, effects of ageing on skin, hyperpigmentation, acne and warts. The devices and methods provided herein may be used in combination with other treatments or medicaments.

### Brief description of the drawings

FIG. 1 is a drawing of a NO delivery device having a pouch in a pouch design;
FIG. 2A and B show a NO delivery device having an integrated double pouch design before and after activation;
FIG. 3 A and B show a NO delivery device having an integrated design with a release liner before and after activation;
FIG. 4 shows top and side cross-section views of a NO delivery device comprising two separable compartments;
FIG. 5 is a perspective view of a double folded release liner.

### Detailed description of the invention

Specific embodiments of the invention are described with reference to the accompanying drawings. This invention may, however, be embodied in many different forms and should not be construed as limited to the embodiments set forth herein; rather, these embodiments are provided so that this disclosure will be thorough and complete, and will fully describe the invention to those skilled in the art. In the drawings, like numbers refer to like elements. Preferred features of each aspect of the invention are as for each of the other aspects mutatis mutandis.

Examples are presented in order to demonstrate the advantages of the present invention over the prior art in order to fully illustrate the preferred embodiments of the invention. The examples herein should in no way be construed, however, as limiting the scope of the invention.

### Chemistry

Nitric oxide (NO) is produced, according to certain embodiments of the present invention, by the reaction of a NO precursor with a reducing agent capable of forming a transient nitrosyl complex. NO precursors include nitrite, nitric acid, and nitrate. In some embodiments, a NO releasing polymer as described in WO 2008/116497, WO 2008/116925 and EP 1871433 can be used as a NO precursor that releases NO when contacted with a proton donor. In these cases, NO eluted from the polymer is initiated by contact with water or other proton donor and the eluted NO forms a complex with a reducing agent/complexing agent or a complexing agent.

Examples of chemical reactions for producing NO include:

3 NaNO₂ + 3 FeSO₄ + 3 H₂O → Fe₂(SO₄)₃ + 3 NaOH + FeOH₃ + 3 NO **(1)**

2 NaN02 + 2 FeS04 + 2 H₂SO₄ → Fe₂(SO₄)₃ + 2 NaHSO₄ + 2 H₂O + 2 NO **(2)**

2 NaNO₂ + 2 KI + 3 H₂SO₄ → I₂ + 4 KHSO₄ + 2 NO **(3)**

8 HNO₃ + 3 Cu → 3 Cu(NO₃)₂ + 4 H₂O + 2NO **(4)**

2 NaNO₂ + 2H⁺ → 2HNO₂ →N₂O₃ + H₂O, N₂O₃ + ascorbic acid →NO + ascorbyl radical **(5)**

NO₂⁻+ H⁺ → HNO₂, NO₂⁻+ HNO₂ → N₂O₃ + OH⁻, N₂O₃ → NO₂ + NO **(6)**.

Preferred Nitrite salts and aqueous nitrite solutions or gels include LiNO₂, NaNO₂, KNO₂, RbNO₂, CsNO₂, FrN02, Be(NO)₂, Mg(NO₂)₂, Ca(N02)2, Sr(N02)2, Ba(N02)2, and Ra(NO₂)₂. Examples of reducing nitrosyl complex forming agents include, but are not limited to, Cu, Cu(I), V(III), Mo(VI), Fe(II), I⁻, Ti(III), Co (II), Mn (II), and Cr(III) and their salts. The NO precursor and reducing agent may be in the form a solid, aqueous solution or gel. Some reducing nitrosyl complex forming agents, such as Fe(II), change color when they form nitrosyl complexes, allowing the activation of NO production and depletion to be monitored.

The above reactions may advantageously take place in an environment excluding atmospheric oxygen. Nitrite (NO₂⁻) or other NO precursor and reducing nitrosyl complex forming agents (CA) are initially present in the reaction in a predetermined ratio (NO₂⁻):(CA), which can be varied so as to result in faster or slower nitric oxide release-rates. If (NO₂⁻) = 1, then (CA), is varied between 1.5 and 10 for NO release to be controlled by complex formation. The presence of an excess of reducing/complexion agent ensures that unreacted reducing/complexing agent remains after NO production to complex with NO. For example, ferrous ion reacts with nitrite to form NO and ferric ion. Ferric ion does not form a transient complex with NO. Unreacted ferrous ion, however, is available because the reducing/complexing agent ferrous sulfate is present in excess. In the case of nitrite and ferrous sulfate, the transient complex formed is [Fe(H₂O)5(NO)]²⁺. The transient NO complexes serve to control the release of NO by delaying NO release from the device in a predictable manner.

The nitrite concentration is preferably between 0.1 and 100 µmol/cm² release area and more preferably from 0.5 to 10 µmol/cm² release area. The reducing/complexing agent may be stabilized by the presence of an acid. Acid is not required for the production of NO from nitrite and a reducing agent, for example, and large changes in absolute and relative acid concentrations of acid in these reactions do not alter the rates of NO release from a NO delivery device according to the present invention. The presence of acid, however, stabilizes reducing agents such as ferrous sulfate, particularly if atmospheric oxygen is present. Acid is not necessary if the device is prepared in a reduced oxygen environment. Acids useful for producing NO and/or stabilizing reducing agents include acetic acid, lactic acid, tartaric acid, ascorbic acid, citric acid salicylic acid, HCl, HBr, H₂SO₄, HNO₃, HClO₄, HI, and combinations thereof. By the use of the methods described herein, it is possible to produce NO delivery devices that produce and release NO containing less than 5% of impurities, less than 2% of impurities, and less than 0.5% of impurities by weight.

### Devices

Nitric oxide delivery devices according to the invention comprise two sealed enclosures, separated a barrier that can be removed, broken, or otherwise breached so that contents of the two compartments can come into contact. In some embodiments, one of the compartments contains a reducing agent/nitrosyl complex forming agent and the other compartment contains nitrite and/or another NO precursor. In other embodiments the reducing agent is not a nitrosyl complex forming agent and a separate NO complexing agent may be present in one of the compartments. The device may have more than two compartments and more than one barrier, if desired, so long as breaking or removing a barrier results in bringing previously isolated NO precursor-containing and reducing nitrosyl complex forming agent containing compartments into fluid contact. Water must be present in at least one compartment of the device to allow mixing and reaction between chemical components. For example, a compartment may contain a solid reducing/complexing agent and a solid nitrite salt on separate portions of an absorbent pad in one compartment and an aqueous solution in another compartment. In this case both NO producing reagents are in the same compartment and separated from an aqueous solution by a breakable or removable barrier. For topical treatment applications, the device is preferably permeable to NO on one side that is configured to face the treatment site and otherwise impermeable to NO so that the NO produced upon mixing of the contents of the two compartments is directed toward the treatment site. The side facing the treatment site preferably comprises a silicone layer or NO permeable adhesive that holds the device in close contact with the treatment site.

The rate of NO release may also be regulated by limiting its release through a NO permeable membrane or film. In some cases it may be sufficient to regulate and prolong NO release by selecting thicknesses of NO permeable membranes, such as those disclosed in WO 2008/116497 and 2008/116925 and EP 1871433, without forming nitrosyl complexes. In such cases, the reducing agent need not be a complex forming agent or, if it does form a nitrosyl complex, it need not be present in excess.

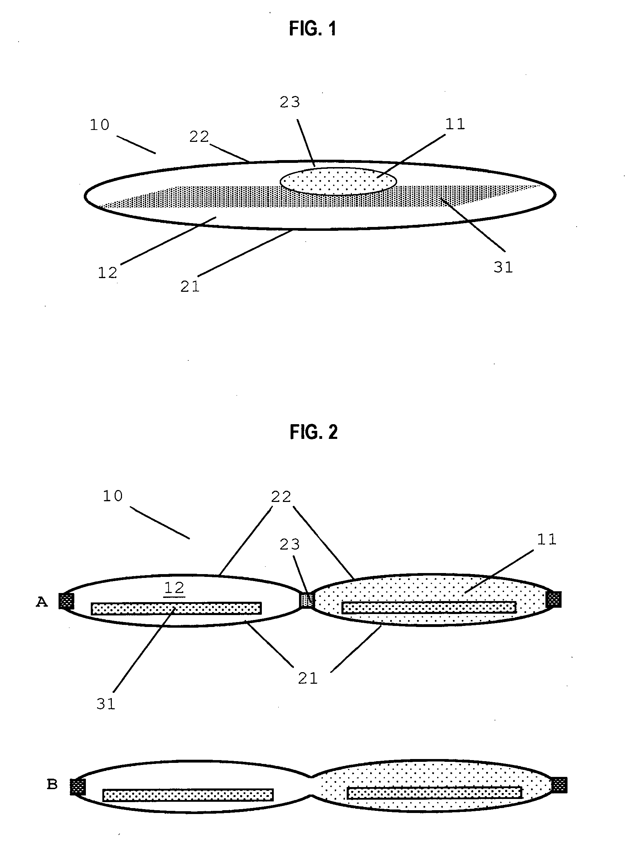

One example of a device according to the invention having a pouch in a pouch configuration is shown in **FIG. 1**. The device **10** comprises a first compartment **11** in the form of a pouch containing a reducing nitrosyl complex forming agent and a second compartment **12** in the form of a pouch containing nitrite. At least one of the compartments also contains water. The first compartment **11** acts as a barrier **23** and is made from a material that allows the pouch to be ruptured by applying pressure, for example by squeezing it between a finger and thumb. The second compartment **12** contains an optional absorbent pad **31** and comprises a first side **21** and a second side **22**, which may be made from the same material or different materials. The first side **21** is made of a NO permeable material, preferably a film or membrane having a predetermined, selected NO permeability and optionally coated with an adhesive that is covered by a removable backing that is impermeable to oxygen. The second side **22** of the second compartment is NO impermeable. This may be accomplished, for example, by covering the second side with a backing material such as aluminum foil that is impervious to NO or by making the second side from a plastic film that is not NO permeable and is preferably translucent or transparent. An absorbent **31** made of cotton, cellulose tissue, or other absorbent material may be included in the second compartment to facilitate an even distribution of NO producing solution after the pouch **11** is ruptured. In some embodiments the absorbent may be an ion exchange membrane that sequesters nitrite ion and thereby slowing the conversion of nitrite to nitric oxide. The locations of the NO precursor and reducing agent may be reversed. One of the two compartments must contain liquid water.

A second example of a device according to the invention having an integrated side-by-side, double pouch configuration is shown in **FIG. 2** **A and B**. The device **10** comprises a first compartment **11** containing a reducing nitrosyl complex forming agent and a second compartment **12** containing a NO precursor. The first compartment **11** and second compartment **12** are both formed from a first film **21** and a second film that are glued or heat welded together in a pattern to form the two compartments. A barrier **23** separating the two compartments is made by making a glued section or welded section that is easier to rupture than the other sections forming the compartments. In the case of glued embodiments, less or weaker glue can be used to form the barrier. In welded embodiments, a lower welding temperature, thinner weld, or shorter welding time may be used for the barrier. The barrier **23** may be ruptured, for example, by squeezing either one of the compartments between a thumb and forefinger. Mixing of the contents may be accelerated by alternately squeezing the two compartments. The first film and second film **22** may be made of the same or different materials. For example, both films may comprise a membrane having a controlled permeability to NO. NO permeability can be controlled, for example, by employing a membrane having a NO permeability that is inversely proportional to membrane thickness and selected a thickness corresponding to a desired permeability. In such a case the second film is coated or covered by a backing material that is impermeable to NO. Alternatively, the first and second films/membranes **21**, **23** may be made of different materials with at least the first membrane being permeable to NO. The second membrane may be made of a material that is permeable to NO and covered with a backing or it made be made from a material that is impermeable to NO. An absorbent may be present in one or both of the compartments. **FIG. 2B** is a side view of the device after being activated by breaking the barrier **23**. The locations of the NO precursor and reducing agent may be reversed. One of the two compartments must contain liquid water.

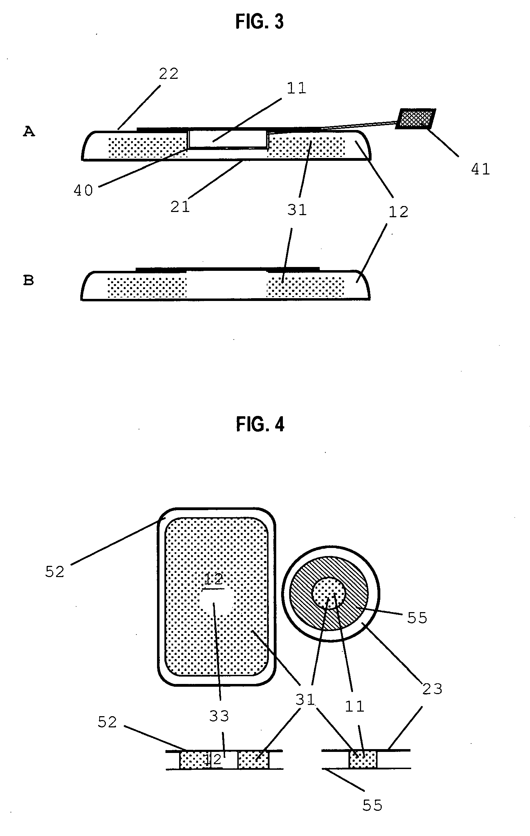

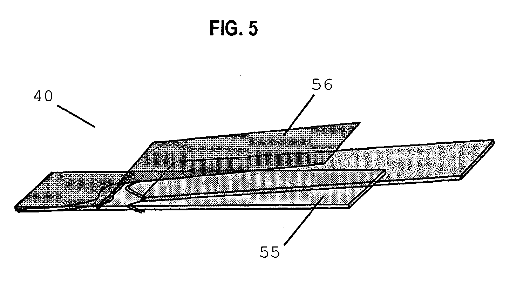

**FIG. 3** **A and B** show side cross-section views of a preferred embodiment of a NO delivery device having an integrated design with a release liner. In this configuration, the first and second compartments **11**,**12** are separated by one or more removable release liners **40** comprising a tab with a grip **41** to facilitate liner removal. A more detailed example of an exemplary release liner is shown in **FIG. 5** that comprises one release liner **56** with an adhesive and one release liner **57** with a silicone treated side laminated together and double folded away from each other at the end. The first compartment **11** extends from the back side of the device **22** into the second compartment **12** so that absorbent pads **31** in both compartments are in apposition to one another. The release liner **40** forms a removable barrier between the first and second compartments **11**, **12**. As an example, the first compartment **11** contains solid sodium nitrite impregnated on the absorbent and the second compartment **12** contains a solution of ferrous sulfate in the absorbent pad. For activation the tab **41** is pulled, releasing the liners in a rolling motion from each side. After removal of the release liner, the absorbent disc of the first compartment moves into the hole of the second compartment. Once the second disc is in place, the device may be repeatedly squeezed to accelerate mixing of the ferrous solution and the sodium nitrite salt. Mixing causes the formation of NO, which is complexed by excess ferrous sulfate to form a green solution. The device is at least partially translucent or transparent, allowing the user to monitor the device for activation and NO production. As NO is depleted, the green color diminishes and eventually disappears, indicating that the device is depleted of NO.

The side **21** of the device to be directed toward the treatment site may coated with silicone or an acceptable adhesive and covered by a removable, oxygen impermeable liner that is preferably not removed until immediately before placing the activated device on a treatment site. The locations of the NO precursor and reducing agent may be reversed. One of the two compartments must contain liquid water.

**FIG. 4** shows a top view of analogous to that shown in **FIG. 3** but embodied as two separate compartments to be joined after the removal of two separate barriers. The second compartment **12** contains an absorbent pad **31** filling much of the compartment except for a disc-shaped area **33**. The top side of the second compartment comprises a NO impermeable film (not visible in top view) having a disc shaped hole aligned with the disc-shaped area **33** lacking the absorbent pad **31**. The hole in the film is covered by a removable liner **52** which, in the embodiment shown, covers the entire top side of the second compartment. The first compartment **11** is substantially filled with an absorbent pad **31** and is sized to fit into the hole in the film of the second compartment **12**. One side of the first compartment comprises a NO impermeable film **55** that is larger in diameter than the first compartment and comprises an adhesive that allows it to seal against the back film of the second compartment around the disc-shaped opening. The other side of the first compartment **11** is covered by a removable liner **23** covering the first compartment **11** and the backing **55**. To activate the device, the liner **52** on the second compartment and the liner **23** of the first compartment are removed and the first compartment **11** is inserted into the disk-shaped hole in the second compartment **12**. The NO impermeable film **55** on the first compartment is pressed against the second compartment to evacuate air from the disc-shaped opening and form a water tight and gas tight seal.

The embodiments shown in **FIGS. 1-4** preferably contain a reducing nitrosyl complex forming agent that changes color when complexed with NO with an intensity of color that corresponds to the concentration of NO in the device. This allows a user to monitor the device for the initial production of NO when the barrier is broken or removed and eventual depletion of NO from the device after extended use. For embodiments in which the production of NO is accompanied by a change in pH and no complexing agent or no color changing complexing agent is present, activation of the device may be monitored using a pH indicator that changes color. The films and membranes used for making the device may be any suitable polymer film or membrane. The films/membranes are preferably hydrophobic and have a controlled thickness and are preferably compatible with gamma ray and other sterilization methods. The films should be impermeable to oxygen and water or components contained within the two compartments, with the exception of the NO permeable membrane which is permeable to NO and oxygen. Backing materials may be selected from any suitable backing material such as polyethylene. Hydrophobic wound dressing materials such as lipid layers are compatible for use in combination with NO delivery devices. The devices need not be held at the treatment site by adhesives and may be incorporated into elastic bandages instead or in addition to using adhesives.

A NO delivery device according to the invention may be in the form of a balloon made of a NO permeable film/membrane at a distal end of a catheter. The balloon is filled with an activated NO producing solution that is formed by pumping an aqueous solution through one or more compartments containing a NO precursor and a reducing/complexing agent. This type of catheter is useful, for example, to prevent bladder infection when using a Foley catheter.

In some cases it is an advantage to prepare NO delivery devices under nitrogen under reduced pressure (e.g. 40% nitrogen and 60% vacuum) to ensure that oxygen is substantially absent from device. For example, if a device contains aqueous ferrous sulfate and no stabilizer, the shelf life of the device may be extended by excluding oxygen from the device and preventing the absorption of atmospheric oxygen into the device. Devices made in this way have shelf lives of at least 3 months at 40°C. In a method of using the device, it is preferred that the side of the device facing the treatment site be covered with a release liner that is impermeable to oxygen until immediately prior to placement on the treatment site to prevent the formation of nitrogen dioxide outside the NO permeable membrane. The inventors have found that exposure of the NO permeable membrane side of the device to atmospheric oxygen may lead to the formation of NO₂ since oxygen enters in to the gas permeable membrane and that this can be avoided by the use of an oxygen impermeable release liner.

In some embodiments, the NO precursor and reducing agent may be dried or absorbed onto different, non-overlapping portions of an absorbent pad and located in the same first or second compartment. The other of the first and second compartments contains water or an aqueous solution that mixes with the NO precursor and reducing agent after the barrier between the compartments is removed or broken. The number of compartments is not necessarily limited to two, although two compartments provides the simplest configuration for most applications.

### Examples

NO delivery devices according to the present invention have been produced that release NO for durations of from below 5 hours to more than 12 hours with NO release rates of between 0.02 and 0.25 µmol-min. NO release profiles range from being strongly time dependent with rapid decrease in release rate over time to maintaining nearly constant NO release over several hours. The devices contain between 30 µmol and 100 µmol NO precursor and release over 90% of NO expected to be produced. The thicknesses of the NO permeable membrane vary from less than 45 µm to more than 100 µm depending on the desired permeability. Total released NO and release profiles are reproducible to within 10% or less. Tables 1 and 2 summarize examples of patch drug delivery devices according to the embodiment shown in **FIGs. 1 and 2**. In addition to the examples provided hereinabove, many other combinations of NO precursors, and ratios of reactants are possible without deviating from the invention.

**Table 1. Pouch in Pouch Examples**

| NO Precursor (P) | Precursor amount | Membrane thickness | Reducing Agent (CA) | Stabilizer (S) | Ratio P:CA:S |
|---|---|---|---|---|---|
| NaNO₂ | 100 µmol | Thin | FeSO₄ | H₂SO₄ | 1:4:4 |
| NaNO₂ | 80 µmol | Thin | FeSO₄ | H₂SO₄ | 1:5:5 |
| NaNO₂ | 100 µmol | Thin | FeSO₄ | H₂SO₄ | 1:5:5 |
| NaNO₂ | 80 µmol | Thin | FeSO₄ | H₂SO₄ | 1:8:5 |
| NaNO₂ | 30 µmol | Thin | FeSO₄ | H₂SO₄ | 1:13:13 |
| NaNO₂ | 80 µmol | Thin | FeSO₄ | H₂SO₄ | 1:2.:2. |
| NaNO₂ | 100 µmol | Thin | FeSO₄ | H₂SO₄ | 1:7:7 |
| NaNO₂ | 120 µmol | Thin | FeSO₄ | H₂SO₄ | 1:5:5 |
| NaNO₂ | 120 µmol | Medium | FeSO₄ | H₂SO₄ | 1:3:3 |
| NaNO₂ | 120 µmol | Thick | FeSO₄ | H₂SO₄ | 1:2:2 |
| NaNO₂ | 30 µmol | Thick | FeSO₄ | H₂SO₄ | 1:13:13 |

**Table 2. Double Pouch Examples *No complex formation.**

| NO Precursor (P) | Precursor amount | Reducing Agent (CA) | Stabilizer (S) | Ratio P:CA:S |
|---|---|---|---|---|
| NaNO₂ | 30 µmol | FeSO₄ | H₂SO₄ | 1:4:4 |
| NaNO₂ | 100 µmol | FeSO₄ | H₂SO₄ | 1:4:2 |
| NaNO₂ | 100 µmol | FeSO₄ | H₂SO₄ | 1:4:1 |
| NaNO₂ | 100 µmol | FeSO₄ | H₂SO₄ | 1:4:0.5 |
| NaNO₂ | 100 µmol | FeSO₄ | - | 1:1:0 |
| NaNO₂ | 100 µmol | FeSO₄ | H₂SO₄ | 1:2:1 |
| NaNO₂ | 100 µmol | FeSO₄ | H₂SO₄ | 1:1:1 |
| NaNO₂ | 100 µmol | Ascorbic Acid* | - | 1:2:0 |
| NaNO₂ | 100 µmol | FeSO₄ | Citric acid | 1:1:1 |
| 3% HNO₃ | 800 µmol | Cu metal | - | 1:2.5:0 |
| 6% HNO₃ | 800 µmol | Cu metal | - | 1:1:0 |

### Methods of Treatment with Devices

Nitric oxide delivery devices may be used in method of treating a variety of diseases and conditions, including conditions that are cosmetic in nature. Such methods generally involve the activation and application of a NO delivery device according to the invention to a treatment site on subject such as a human or other mammal or animal. The NO release profile, including duration and rate of NO release may be controlled by the appropriate selection of relative and absolute amounts of nitrite, acid, and complex forming agent, NO permeable membrane composition and thickness, and device dimensions. Methods according to the present invention method for preventing and/or treating pain, ulcers, neuropathies, wounds, infections, cardiovascular conditions and/or diseases, cosmetic conditions and/or sequelae, or a combination thereof.

As used herein, the singular forms "a", "an" and "the" are intended to include the plural forms as well, unless expressly stated otherwise. It will be further understood that the terms "includes," "comprises," "including" and/or "comprising," when used in this specification, specify the presence of stated features, integers, steps, operations, elements, and/or components, but do not preclude the presence or addition of one or more other features, integers, steps, operations, elements, components, and/or groups thereof. It will be understood that when an element is referred to as being "connected" or "coupled" to another element, it can be directly connected or coupled to the other element or intervening elements may be present. As used herein, the term "and/or" includes any and all combinations of one or more of the associated listed items.

The above description focuses on an embodiment of the present invention applicable to a topical patch releasing NO. However, it will be appreciated that the invention is not limited to this application but may be applied to many other delivery devices, including for example wraps, bandages, etc.

The present invention has been described above with reference to specific embodiments. However, other embodiments than the above described are equally possible within the scope of the invention. Different method steps than those described above, performing the method by hardware or software, may be provided within the scope of the invention. The different features and steps of the invention may be combined in other combinations than those described. The scope of the invention is only limited by the appended patent claims.

## Claims

1. A nitric oxide delivery device comprising an enclosure, said enclosure forming or containing:
a first compartment containing an amount of a reducing agent and an amount of an agent capable of forming a transient complex with nitric oxide;
a second compartment containing an amount of a nitric oxide precursor that forms nitric oxide when contacted with and reduced by the reducing agent;
a barrier separating the contents of the first and second compartments
wherein:
said enclosure comprises a nitric oxide-permeable membrane that is impermeable to the nitric oxide precursor, the agent capable of forming a transient complex with nitric oxide, and the reducing agent:
at least one of the first compartment and the second compartment contains water;
the first and second compartments are configured such that breaking or removing the barrier allows the nitric oxide precursor to mix with the reducing agent and complex forming agent, thereby producing an aqueous nitric oxide producing mixture containing a transient nitrosyl complex;
the agent capable of forming a nitrosyl complex and the nitric oxide precursor are present in the device in a molar ratio in the range of from 1.5:1 to 10:1;
the reducing agent or agent capable of forming a nitrosyl complex or both are selected from the group comprising Fe(II), and salts thereof; and
said transient nitrosyl complex is arranged in said device to delay the release of NO from the device through the nitric oxide-permeable membrane.

2. The device according to claim 1, wherein the reducing agent and the agent capable of forming a nitrosyl complex are the same agent.

3. The device according to claim 1 or 2, wherein the nitric oxide permeable membrane is rate controlling for the release of nitric oxide from the device at some time after the production of nitric oxide is initiated.

4. The device according to any of claims 1-3, wherein air is absent from the first and second compartments.

5. The device according to any of claims 1-4, wherein the nitric oxide precursor is a nitrite salt in the form of a solid, an aqueous solution, or a gel.

6. The device according to claim 5, wherein the nitric oxide precursor comprises one or more of the group consisting of LiNO₂, NaNO₂, KNO₂, RbNO₂, CsNO₂, FrNO₂, Be(NO)₂, Mg(NO₂)₂, Ca(N02)2, Sr(NO₂)₂, Ba(N02)2, Ra(N02)2, and combinations thereof.

7. The device according to any of the preceding claims, wherein the reducing agent and the agent capable of forming a nitrosyl complex is ferrous sulfate.

8. The device according to any of the preceding claims, wherein the agent capable of forming a nitrosyl complex has a different color than the nitrosyl complex it forms.

9. The device according to any of the preceding claims, and further comprising an acid in the first compartment.

10. The device according to claim 9, wherein the acid is selected from the group consisting of acetic acid, lactic acid, tartaric acid, ascorbic acid, citric acid salicylic acid, HCl, HBr, H₂SO₄, HNO₃, HClO₄, HI, and combinations thereof.

11. The device according to any of the preceding claims, wherein the nitric oxide-permeable membrane is located on a first side of the device and a membrane impermeable to nitric oxide is located a second side of the device and the device is configured to be placed on a treatment site of a subject with the first side of the device oriented toward the treatment site and the second side of the device oriented away from the treatment site.

12. The device according to claim 11, wherein the first side of the device comprises an exterior soft silicone layer or adhesive.

13. The device according to claim 11, wherein the barrier is a removable barrier comprising a first release liner covering an opening in the first compartment and a second release liner covering an opening in the second compartment.

14. The device according to claim 13, wherein the first and second compartments are separable before activation of nitric oxide production.

15. The device according to any of the preceding claims, wherein the first and second compartments are pouches and one of the first and second compartments contains the other of said first and second compartments.

16. The device according to any of the preceding claims, wherein one or both of the first and second compartments contains an absorbent.

17. The device according to any of the preceding claims, wherein the nitric oxide precursor comprises nitrite immobilized on an anion exchange material capable of reversibly binding nitrite.

18. The device according to any of the preceding claims, wherein the barrier is a removable release liner.

19. A device according to any of claims 1-18 for use in treating or preventing a disease or condition in an animal or human subject.

20. The device of claim 19, wherein said disease or condition is selected from the group consisting of pain, an ulcer, neuropathy, a wound, a wart, acne, an infection, a circulatory impairment, a cosmetic condition, sequelae, and combinations thereof.

## Patentansprüche

1. Vorrichtung zur Abgabe von Stickoxid, die eine Umhausung umfasst, wobei die Umhausung bildet oder enthält:
erstes Fach, welches eine Menge eines Reduktionsmittels und eine Menge eines Wirkstoffes enthält, welcher einen transienten Komplex mit Stickoxid bilden kann;
zweites Fach, welches eine Menge einer Stickoxid-Vorläufersubstanz enthält, die Stickoxid bildet, wenn sie mit dem Reduktionsmittel in Kontakt kommt und von diesem reduziert wird;
eine Schranke, welche die Inhalte des ersten und des zweiten Faches voneinander trennt,
wobei:
diese Umhausung eine stickoxiddurchlässige Membran umfasst, welche für die Stickoxid-Vorläufersubstanz, den Wirkstoff, der einen transienten Komplex mit Stickstoff bilden kann, sowie das Reduktionsmittel undurchlässig ist:
wobei mindestens eines aus erstem Fach und zweitem Fach Wasser enthält;
das erste und das zweite Fach so eingerichtet sind, dass ein Brechen oder Entfernen der Schranke eine Vermischung der Stickoxid-Vorläufersubstanz mit dem Reduktionsmittel sowie dem Komplexbildungs-Wirkstoff erlaubt, wodurch eine wässrige stickoxiderzeugende Mischung erzeugt wird, welche einen transienten Nitrosylkomplex enthält;
der Wirkstoff, der einen Nitrosylkomplex bilden kann, und die Stickoxid-Vorläufersubstanz in der Vorrichtung in einem Molarverhältnis vorliegen, das im Bereich von 1,5: 1 bis 10:1 liegt;
das Reduktionsmittel oder der Wirkstoff, der einen Nitrosylkomplex bilden kann, oder beide aus einer Gruppe ausgewählt werden, welche Fe(II) und Salze von diesem umfasst; und
der transiente Nitrosylkomplex so in der Vorrichtung angeordnet ist, dass er die Abgabe von NO aus der Vorrichtung durch die stickoxiddurchlässige Membran verzögert.

2. Vorrichtung gemäß Anspruch 1, wobei es sich bei dem Reduktionsmittel und dem Wirkstoff, der einen Nitrosylkomplex bilden kann, um denselben Wirkstoff handelt.

3. Vorrichtung gemäß Anspruch 1 oder 2, wobei die stickoxidurchlässige Membran zu einem Zeitpunkt, nachdem die Produktion von Stickoxid initiiert worden ist, eine Rate Control für die Abgabe von Stickoxid aus der Vorrichtung bewirkt.

4. Vorrichtung gemäß einem beliebigen der Ansprüche 1 - 3, wobei in dem ersten und zweiten Fach keine Luft vorhanden ist.

5. Vorrichtung gemäß einem beliebigen der Ansprüche 1 - 4, wobei es sich bei der Stickoxid-Vorläufersubstanz um ein Nitritsalz in Form eines Feststoffes, einer wässrigen Lösung oder eines Gels handelt.

6. Vorrichtung gemäß Anspruch 5, wobei die Stickoxid-Vorläufersubstanz eines oder mehrere aus der Gruppe umfasst, welche besteht aus: LiNO₂, NaNO₂, KNO₂, RbNO₂, CsNO₂, FrNO₂, Be(NO)₂, Mg(NO₂)₂, Ca(NO₂)₂, Sr(NO₂)₂, Ba(NO₂)_{2,} Ra(NO₂)₂ und Kombinationen von diesen.

7. Vorrichtung gemäß einem beliebigen der vorangegangenen Ansprüche, wobei es sich bei dem Reduktionsmittel und dem Wirkstoff, welcher einen Nitrosylkomplex bilden kann, um Eisensulfat handelt.

8. Vorrichtung gemäß einem beliebigen der vorangegangenen Ansprüche, wobei der Wirkstoff, welcher einen Nitrosylkomplex bilden kann, eine andere Farbe hat als der Nitrosylkomplex, den er bildet.

9. Vorrichtung gemäß einem beliebigen der vorangegangenen Ansprüche, und ferner eine Säure im ersten Fach umfassend.

10. Vorrichtung gemäß Anspruch 9, wobei die Säure aus der Gruppe ausgewählt wird, die aus Essigsäure, Milchsäure, Weinsäure, Ascorbinsäure, Zitronensäure, Salicylsäure, HCl, HBr, H₂SO₄, HNO₃, HCIO₄, HI, und Kombinationen von diesen besteht.

11. Vorrichtung gemäß einem beliebigen der vorangegangenen Ansprüche, wobei die stickoxidurchlässige Membran sich auf einer ersten Seite der Vorrichtung befindet und sich eine Membran, die undurchlässig für Stickoxid ist, auf einer zweiten Seite der Vorrichtung befindet, und wobei die Vorrichtung so eingerichtet ist, dass sie auf einer Behandlungsstelle bei einer Person platziert werden kann, wobei die erste Seite der Vorrichtung zu der Behandlungsstelle und die zweite Seite der Vorrichtung von der Behandlungsstelle weg ausgerichtet ist.

12. Vorrichtung gemäß Anspruch 11, wobei die erste Seite der Vorrichtung eine äußere weiche Silikonschicht oder ein Klebemittel umfasst.

13. Vorrichtung gemäß Anspruch 11, wobei es sich bei der Schranke um eine abnehmbare Schranke handelt, die einen ersten Release Liner umfasst, welcher eine Öffnung in dem ersten Fach abdeckt, sowie einen zweiten Release Liner umfasst, welcher eine Öffnung in dem zweiten Fach abdeckt.

14. Vorrichtung gemäß Anspruch 13, wobei sich das erste und das zweite Fach voneinander trennen lassen, bevor die Aktivierung der Stickoxidproduktion erfolgt.

15. Vorrichtung gemäß einem beliebigen der vorangegangenen Ansprüche, wobei es sich bei dem ersten und dem zweiten Fach um Beutel handelt und eines aus erstem und zweitem Fach das jeweils andere aus erstem und zweitem Fach enthält.

16. Vorrichtung gemäß einem beliebigen der vorangegangenen Ansprüche, wobei eines oder beide aus erstem und zweitem Fach ein Absorptionsmittel enthalten.

17. Vorrichtung gemäß einem beliebigen der vorangegangenen Ansprüche, wobei die Stickoxid-Vorläufersubstanz ein Nitrit enthält, das auf einem Anionenaustausch-Material fixiert ist, welches Nitrit reversibel binden kann.

18. Vorrichtung gemäß einem beliebigen der vorangegangenen Ansprüche, wobei es sich bei der Schranke um einen abnehmbaren Release Liner handelt.

19. Vorrichtung gemäß einem beliebigen der Ansprüche 1 - 18 zur Behandlung oder Vorbeugung einer Krankheit oder eines Leidens bei einem Tier oder einem Menschen.

20. Vorrichtung gemäß Anspruch 19, wobei diese Krankheit oder dieses Leiden aus einer Gruppe ausgewählt ist, zu welcher Schmerzen, ein Geschwür, Neuropathie, eine Wunde, eine Warze, Akne, eine Infektion, eine Kreislaufstörung, ein kosmetisches Leiden, Folgekrankheiten, oder Kombinationen von diesen gehören.

## Revendications

1. Dispositif destiné à fournir de l'oxyde nitrique comprenant une enceinte, ladite enceinte formant ou contenant :
un premier compartiment contenant une quantité d'un agent réducteur et une quantité d'un agent capable de former un complexe transitoire avec de l'oxyde nitrique,
un deuxième compartiment contenant une quantité de précurseur d'oxyde nitrique qui forme de l'oxyde nitrique lorsqu'il est en contact avec l'agent réducteur et réduit par celui-ci,
une barrière séparant les contenus des premier et deuxième compartiments
dans lequel :
ladite enceinte comprend une membrane perméable à l'oxyde nitrique qui est imperméable au précurseur d'oxyde nitrique, à l'agent capable de former un complexe transitoire avec l'oxyde nitrique, et à l'agent réducteur :
au moins l'un du premier compartiment et du deuxième compartiment contient de l'eau,
les premier et deuxième compartiments sont configurés de sorte que la rupture ou le retrait de la barrière permet au précurseur d'oxyde nitrique de se mélanger avec l'agent réducteur et l'agent formant un complexe, en produisant de cette manière un mélange produisant de l'oxyde nitrique aqueux contenant un complexe de nitrosyle transitoire,
l'agent capable de former un complexe de nitrosyle et le précurseur d'oxyde nitrique sont présents dans le dispositif suivant un rapport molaire dans la plage de 1,5:1 à 10:1,
l'agent réducteur ou l'agent capable de former un complexe de nitrosyle ou les deux sont sélectionnés à partir du groupe comprenant Fe(II), et ses sels, et
ledit complexe de nitrosyle transitoire est placé dans ledit dispositif pour retarder la libération de NO à partir du dispositif à travers la membrane perméable à l'oxyde nitrique.

2. Dispositif selon la revendication 1, dans lequel l'agent réducteur et l'agent capable de former un complexe de nitrosyle sont le même agent.

3. Dispositif selon la revendication 1 ou 2, dans lequel la membrane perméable à l'oxyde nitrique contrôle le débit de la libération d'oxyde nitrique à partir du dispositif un certain temps après le début de la production d'oxyde nitrique.

4. Dispositif selon l'une quelconque des revendications 1 à 3, dans lequel l'air est absent des premier et deuxième compartiments.

5. Dispositif selon l'une quelconque des revendications 1 à 4, dans lequel le précurseur d'oxyde nitrique est un sel de nitrite sous la forme d'un solide, d'une solution aqueuse, ou d'un gel.

6. Dispositif selon la revendication 5, dans lequel le précurseur d'oxyde nitrique comprend un ou plusieurs des groupes parmi LiNO₂, NaNO₂, KNO₂, RbNO₂, CsNO₂, FrNO₂, Be(NO₂), Mg(NO₂)₂, Ca(NO₂)₂, Sr(NO₂)₂, Ba(NO₂)₂, Ra(NO₂)₂, et des combinaisons de ceux-ci.

7. Dispositif selon l'une quelconque des revendications précédentes, dans lequel l'agent réducteur et l'agent capable de former un complexe de nitrosyle sont le sulfate ferreux.

8. Dispositif selon l'une quelconque des revendications précédentes, dans lequel l'agent capable de former un complexe de nitrosyle a une couleur différente de celle du complexe de nitrosyle qu'il forme.

9. Dispositif selon l'une quelconque des revendications précédentes, et comprenant en outre un acide dans le premier compartiment.

10. Dispositif selon la revendication 9, dans lequel l'acide est sélectionné à partir du groupe incluant l'acide acétique, l'acide lactique, l'acide tartrique, l'acide ascorbique, l'acide citrique, l'acide salicylique, HCl, HBr, H₂SO₄, HNO₃, HClO₄, Hl, et des combinaisons de ceux-ci.

11. Dispositif selon l'une quelconque des revendications précédentes, dans lequel la membrane perméable à l'oxyde nitrique est située d'un premier côté du dispositif et une membrane imperméable à l'oxyde nitrique est située d'un deuxième côté du dispositif et le dispositif est configuré pour être placé sur un site de traitement d'un sujet avec le premier côté du dispositif orienté en direction du site de traitement et le deuxième côté du dispositif orienté à l'opposé du site de traitement.

12. Dispositif selon la revendication 11, dans lequel le premier côté du dispositif comprend une couche de silicone molle ou un adhésif.

13. Dispositif selon la revendication 11, dans lequel la barrière est une barrière amovible comprenant un premier revêtement libérable recouvrant une ouverture dans le premier compartiment et un deuxième revêtement libérable recouvrant une ouverture dans le deuxième compartiment.

14. Dispositif selon la revendication 13, dans lequel les premier et deuxième compartiments sont séparables avant activation de la production d'oxyde nitrique.

15. Dispositif selon l'une quelconque des revendications précédentes, dans lequel les premier et deuxième compartiments sont des poches et un premier des premier et deuxième compartiments contient l'autre des premier et deuxième compartiments.

16. Dispositif selon l'une quelconque des revendications précédentes, dans lequel l'un ou les deux des premier et deuxième compartiments contiennent un absorbant.

17. Dispositif selon l'une quelconque des revendications précédentes, dans lequel le précurseur d'oxyde nitrique comprend un nitrite immobilisé sur un matériau d'échange d'anions capable de se lier de manière réversible avec le nitrite.

18. Dispositif selon l'une quelconque des revendications précédentes, dans lequel la barrière est un revêtement libérable amovible.

19. Dispositif selon l'une quelconque des revendications 1 à 18 destiné à être utilisé pour traiter ou empêcher une maladie ou un état chez un sujet animal ou humain.

20. Dispositif selon la revendication 19, dans lequel ladite maladie ou ledit état est sélectionné à partir du groupe incluant une douleur, un ulcère, une neuropathie, une blessure, une verrue, l'acné, une infection, une détérioration circulatoire, un état cosmétique, des séquelles, et des combinaisons de ceux-ci.
